Europäisches Patentamt

European Patent Office

Office européen des brevets

(11) Publication number: **0 327 231**
**A1**

## (12) EUROPEAN PATENT APPLICATION

(21) Application number: **89300602.3**

(22) Date of filing: **23.01.89**

(51) Int. Cl.4: **C07C 119/045 , C07C 119/042 , C07C 118/00**

(30) Priority: **05.02.88 GB 8802674**

(43) Date of publication of application:
**09.08.89 Bulletin 89/32**

(84) Designated Contracting States:
**AT BE CH DE ES FR GB GR IT LI NL SE**

(71) Applicant: **IMPERIAL CHEMICAL INDUSTRIES PLC**
**Imperial Chemical House Millbank**
**London SW1P 3JF(GB)**

Applicant: **ICI AMERICAS INC.**
**Concord Pike & New Murphy Road**
**Wilmington Delaware 19897(US)**

(72) Inventor: **Thorpe, David**
**Sterrebeeksesteenweg 45**
**B-3078 Everberg(BE)**
Inventor: **Smith, Richard Colin**
**23 Wilderness Way**
**Chadds Ford, PA 19317(US)**

(74) Representative: **Baken, Philippus Johannes L. H. et al**
**Imperial Chemical Industries PLC Legal Department: Patents P.O. Box 6 Bessemer Road**
**Welwyn Garden City Herts. AL7 1HD(GB)**

(54) **Process for making aliphatic and cycloaliphatic polyisocyanates.**

(57) A method for the preparation of an aliphatic or cycloaliphatic polyisocyanate which comprises heating the product obtained by reacting an aliphatic or cycloaliphatic polyamine having at least two primary amino groups with an excess of an aromatic polyisocyanate having a boiling point at least 20° C higher than the point of the aliphatic or cycloaliphatic polyisocyanate derived from above mentioned polyamines.

This invention relates to a process for the preparation of aliphatic and cycloaliphatic polyisocyanates.

Organic polyisocyanates are conventionally manufactured in bulk by reacting phosgene with organic polyamines. Thus, tolylene diisocyanates are made by phosgenating tolylene diamines and hexamethylene diisocyanate is made by the phosgenation of hexamethylene diamine.

The phosgenation method is generally satisfactory for the production of the high volume aromatic polyisocyanates such as MDI and TDI but for some of the lower volume materials, such as the aliphatic and cycloaliphatic diisocyanates, it would sometimes be convenient to have a practicable process avoiding the use of phosgene. Other methods of making polyisocyanates are known but have generally not been regarded as suitable for commercial exploitation.

It is known from United Kingdom Patent Specification No. 1030305 that organic monoisocyanates are formed as byproducts of the process for preparing biuret polyisocyanates which comprises reacting an organic di- or polyisocyanate with an organic primary monoamine in such proportions that the ratio of -NCO to $NH_2$ is at least 3 and heating the reaction mixture to an elevated temperature.

The present invention provides a method for the preparation of an aliphatic or cycloaliphatic polyisocyanate which comprises heating the product obtained by reacting an aliphatic or cycloaliphatic polyamine having at least two primary amino groups with an excess of an aromatic polyisocyanate having a boiling point at least 20° C higher than the point of the aliphatic or cycloaliphatic polyisocyanate derived from above mentioned polyamines.

Any aliphatic or cycloaliphatic polyamine may be used in the method of the invention provided the derived polyisocyanate has a boiling point at least 20° C lower than that of the aromatic polyisocyanate being used. Such polyamines generally contain from 2 to 12 carbon atoms. As examples of such polyamines there may be mentioned ethylene diamine, 1,5- and 1,6-hexane diamines and isophorone diamine.

Aromatic polyisocyanates which may be used include those which are commercially available such as tolylene diisocyanate (including TDI residues), diphenylmethane diisocyanate and polymethylene polyphenylene polyisocyanates (crude MDI).

The excess of aromatic polyisocyanate relative to the aliphatic or cycloaliphatic polyamine is suitably such that the reaction mixture contains at least 3 moles of polyisocyanate per mole of polyamine.

If desired, the reaction mixture may also contain a liquid of very high boiling point as an inert carrier.

In performing the method of the invention, the product obtained by reacting the aromatic polyisocyanate and aliphatic or cycloaliphatic polyamine is suitably heated to a temperature of at least 120° C. It is particularly convenient to react the aromatic polyisocyanate and the polyamine at an elevated temperature and to remove the aliphatic or cycloaliphatic polyisocyanate from the reaction mixture continuously as it is formed.

The invention is illustrated but not limited by the following Examples :

Example 1

A polymethylene polyphenylene polyisocyanate composition containing approximately 62 % of diisocyanate (400 g) was rapidly stirred at 120° C in a 2 liter glass flask. Isophorone diamine (6 g) was added dropwise over 20 minutes and the resulting mobile mixture containing large solid particles was heated to 180° C for 3 hours. After cooling to room temperature, a mobile liquid containing a dispersion of solids was obtained. This product was fed to a wiped wall thin film evaporator at a wall temperature of 196° C and a pressure of 6 mm Hg.

The distillate obtained (2.7 g) was found to contain 60 % of isophorone diisocyanate and 40 % of mixed isomers of diphenylmethane diisocyanate.

Example 2

A polymeric MDI composition as used in Example 1 (1023 g) was stirred at 150° C and a mixture of 1,6-hexane diamine (20.9 g) and chlorobenzene (50 g) was added dropwise over 1 hour, producing a low viscosity liquid containing large gelatinous lumps. On heating further to 195° C, the lumps dissolved to give a clear solution which was fed dropwise to a wiped wall thin film evaporator at a wall temperature of 196° C and a pressure of 6 mm Hg. A distillate of 47 g was recovered. Chlorobenzene was removed from the distillate in a Rotavapor at 30 mm Hg pressure leaving 13 g of involatile liquid residue which was found to contain 40 % of hexamethylene diisocyanate and 60 % of mixed diphenylmethane diisocyanate isomers.

## Claims

1. A method for the preparation of an aliphatic or cycloaliphatic polyisocyanate which comprises heating the product obtained by reacting an aliphatic or cycloaliphatic polyamine having at least two primary amino groups with an excess of an aromatic polyisocyanate having a boiling point at least 20° C higher than the point of the aliphatic or cycloaliphatic polyisocyanate derived from above mentioned polyamines.

2. A method according to claim 1 wherein the aliphatic or cycloaliphatic polyamine is a hexane diamine or isophorone diamine.

3. A method according to claim 1 or claim 2 wherein the aromatic polyisocyanate is tolylene diisocyanate, diphenylmethane diisocyanate or a polymethylene polyphenylene polyisocyanate or TDI distillation residues.

4. A method to any of claims 1 to 3 using at least 3 moles of aromatic polyisocyanate per mole of polyamine.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,A | GB-A-1 030 305 (FARBENFABRIKEN BAYER) <br> * Claims; examples * | 1-4 | C 07 C 119/045 <br> C 07 C 119/042 <br> C 07 C 118/00 |
|  |  |  | **TECHNICAL FIELDS SEARCHED (Int. Cl.4)** |
|  |  |  | C 07 C 118/00 <br> C 07 C 119/00 |

The present search report has been drawn up for all claims

| Place of search | Date of completion of the search | Examiner |
|---|---|---|
| THE HAGUE | 09-05-1989 | WRIGHT M.W. |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P0401)